# EUROPEAN PATENT APPLICATION

(11) **EP 0 751 215 A2**
(43) Date of publication of application: **02.01.1997**
(21) Application number: 96304760.0
(22) Date of filing: 27.06.1996
(51) Int. Cl.: C12M 1/20, C12M 1/22

(54) **Culture vessel for use with coverslips**

(30) Priority: 29.06.1995 US 496593
(71) Applicant: Becton Dickinson and Company, Franklin Lakes, New Jersey 07417-1880 (US)
(72) Inventor: Grushkin-Lerner, Leslie S., Bedford, MA 01730 (US)
(74) Representative: Ruffles, Graham Keith

(57) **Abstract**

A vessel of the present invention for culturing cells includes an open receptacle with a bottom surface. The bottom surface of the receptacle has a multiplicity of protuberances projecting upwardly from the surface. The upward protuberances serve to support coverslips above the bottom surface of the receptacle allowing substantially easier manipulation of the coverslips when removing the coverslips by substantially preventing adhesion due to surface tension effects of medium and cells.

## Description

### Field of Invention:

The present invention generally relates to cell culture devices and more particularly to a culture vessel with provisions for supporting a coverslip above the bottom.

### Background:

In biological research, small scale cell culture is often conducted on the surface of microscope slide coverslips. Coverslips are readily available, easily cleaned and uniform. Coverslips are commonly made from glass and plastic. The coverslips are available as circles with diameter of 12 millimeters (mm), 18mm, squares with 18mm, 22mm and 25mm sides and rectangles dimensioned from 11mm by 22mm to 48mm by 60mm. When used for cell culture, the coverslips are generally cleaned, coated with protein, dried and placed in a culture vessel having a flat bottom. The culture vessels used for these procedures are either Petri dishes, where several coverslips may be placed at one time, spaced apart from each other, or a multi-well plate where each coverslip is placed in an individual well.

A reference text entitled: Culturing Nerve Cells, G. Banker and K Goslin, MIT Press, (1991) discusses the use of coverslips for cell culture. In the reference, the use of coverslips is reported in a "Protocol for Preparing Low-Density Hippocampal Cultures" on pages 255-259. The "protocol" reports that it is difficult to apply microscopic techniques to cells grown directly in plastic culture dishes. The "protocol" suggests plating hippocampal cells onto glass coverslips treated with a protein, polylysine, to enhance cell adhesion.

According to the "protocol," to conduct a cell mono-culture, i. e., growing only one type of cells, protein coated coverslips are placed flat on the bottom of a container, generally a Petri dish or a well of a multiwell plate, and a cell suspension in an aqueous medium is added to the container. The container with the coverslip and the suspension is incubated to develop a substantially confluent monolayer of the cells on the coverslip. For microscopic analysis of the cells or for further treatment of the cells, the coverslip with the monolayer of cells generally must be removed from the container with forceps. Since both the coverslip and the container bottoms are substantially flat, the aqueous medium often causes the coverslip to adhere strongly to the bottom of the container. Because of this adhesion, handling the coverslip with forceps often cracks the coverslip, disrupts the cells and may render the cells on the coverslip useless for further work.

When a cell co-culture is conducted, i. e., growing cells of a first type in close proximity to cells of a second type so that they interact chemically without direct contact, the coverslip and container technique also is useful. In the "protocol" from the reference Culturing Nerve Cells, the authors suggest applying small dots of wax to the edges of a coverslip prior to coating the coverslip with protein. To prepare a co-culture according to the "protocol," cells of a first type are cultured on the coverslip, on the side with the wax dots, following the mono-culture procedure to a confluent monolayer. The need to culture the first type cells on the side of the coverslip with the wax dots continues the possibility that some coverslips may crack in the handling necessary for the next step. Then, the coverslip with the first type cells is removed from its original container and placed in a second container with a confluent monolayer of cells of a second type cultured on its bottom surface so that the coverslip side having the first type cells faces the second type cells in the second container. In the placement of the coverslip in the second container, the wax dots support the coverslip with the first type of cells on the surface in close proximity to the cells of the second type. In this co-culture application using coverslips, the wax dots provide a separation between the coverslip and the container bottom. In addition to allowing the co-culture to proceed without direct contact between the cells, the wax dots allow the coverslip to be easily picked up and manipulated with forceps by substantially preventing adherence of the coverslip to the bottom of the container.

The addition of wax dots to a coverslip, while providing a separation between the coverslip and the container bottom, is time consuming and technique sensitive. Additionally, the wax dots are a potential source of contamination and may come off during handling. If a container were available that would support a coverslip above its bottom, the efficiency and effectiveness of laboratory work in cell mono-culture and co-culture would be enhanced. Such a container is described below.

### Summary

A vessel of the present invention for culturing cells includes an open receptacle with a bottom surface. The bottom surface of the receptacle has a multiplicity of protuberances projecting upwardly from the surface.

The vessel of the present invention provides a practitioner of cell mono-culture and co-culture a more efficient usage of coverslips for culturing cells. When used in a mono-culture, the protuberances support the coverslips above the bottom allowing easier manipulation of the coverslips, substantially reducing incidence of coverslip breakage due to their sticking to the vessel bottom. Additionally, for co-culture usage, the protuberances support coverslips having a first population of cells in close proximity to a second population of cells grown on the bottom of the vessel without direct contact between the cell populations. The coverslip with the first population then is easily removed from the vessel for study.

### Brief Description of the Drawings

Fig. 1 is an exploded perspective view of a vessel of the present invention;
Fig. 2 is a cross-sectional view of the vessel of the vessel of Fig. 1 along the line 2-2;
Fig. 3 is a schematic cross-sectional view, similar to the view of Fig. 2, of a vessel analogous to the vessel of Fig. I illustrating several protuberance shapes;
Fig. 4 is a perspective view of an embodiment of the vessel of Fig. 1 illustrating manipulation of a coverslip;
Fig. 5 is a top plan view of an embodiment of the vessel of Fig. 1 illustrating placement of several coverslips; and
Fig. 6 is a perspective view of an embodiment of the present invention with a plurality of vessels as shown in Fig. 1 incorporated into a multi-well plate.

### Detailed Description

While this invention is satisfied by embodiments in many different forms, there is shown in the drawings and will herein be described, several embodiments of the invention with the understanding that the present disclosure is to be considered descriptive of the principles of the present invention and is not intended to limit the scope of the invention to the embodiments illustrated. The scope of the invention is measured by the appended claims and their equivalents.

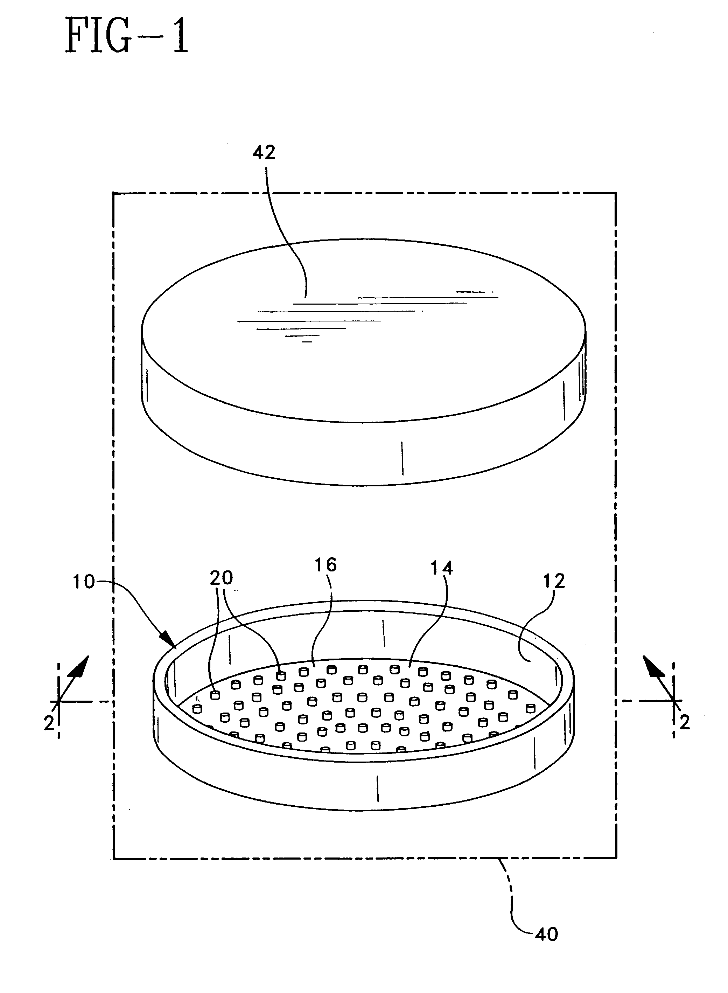

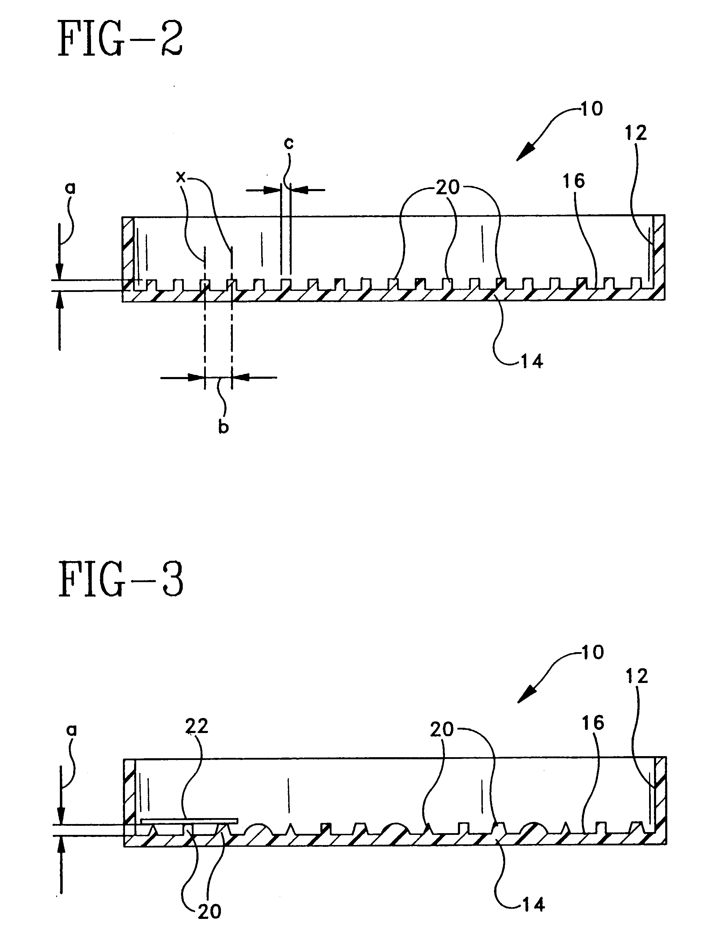

Referring to Figs. 1 and 2, a vessel 10 of the present invention for culturing cells includes an open receptacle 12 with a bottom 14 having a surface 16 with a multiplicity of protuberances 20 projecting upwardly a distance "a" therefrom and spaced apart a distance "b." Preferably, surface 16 is substantially flat, and the projection distance "a" of protuberances 20 is preferably substantially uniform, so that when a coverslip 22 is placed on the bottom of receptacle 12 as shown in Figs. 3, 4 and 5, it is supported substantially parallel to bottom surface 16.

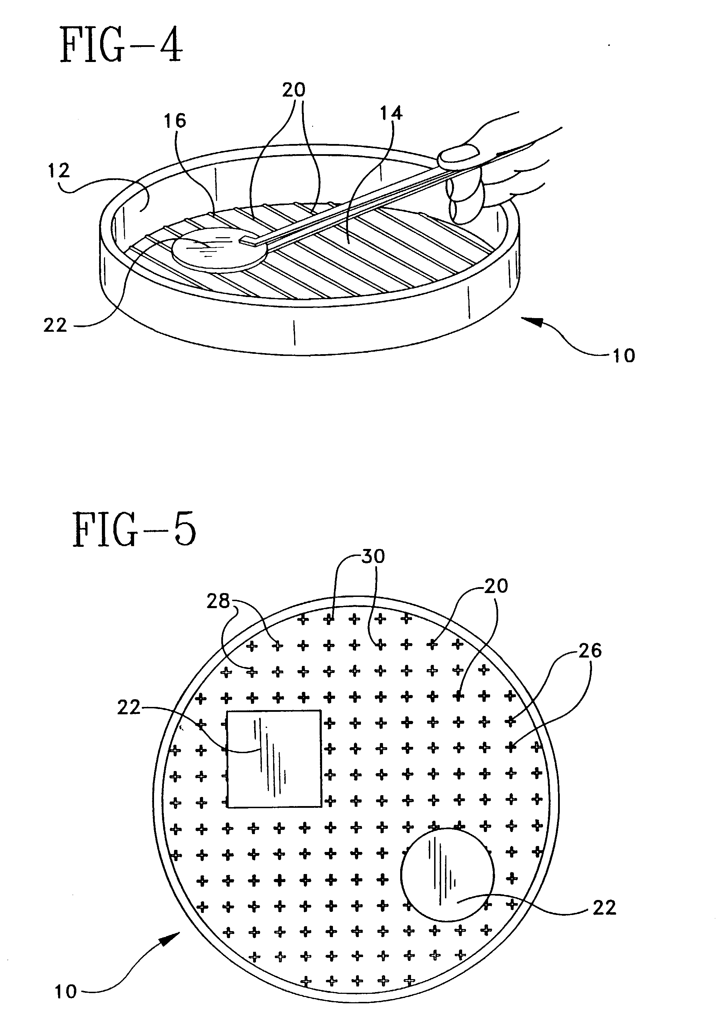

As shown schematically in cross-section in Fig. 3, protuberances 20 may have a variety of geometric shapes including, but not limited to cones, frustrums of cones, cylinders, hemispheres, cubes and the like. Alternatively, as shown in Fig. 4, protuberances 20 may be substantially parallel ridges 24 with a spacing "d" between the ridges. In the case where the protuberances are ridges, dimension "d" preferably is between about 3mm to about 6mm and the ridges are between about lmrn to about 5mm high and between about 0.5 to about 1.5mm wide. For particular applications, taller ridges, wider ridges or with other spacings are considered to be within the scope of the present invention.

Referring to Fig. 5, protuberances 20 may also be in the shape of a cross 26 formed by a first plurality of substantially parallel segmented ridges 28 intersected by a second plurality of substantially parallel segmented ridges 30. Other configurations are within the purview of the invention. For example, the ridges may be a series of spaced-apart, concentric rings, with or without open segments, formed in the bottom of the receptacle. Preferably, as shown in Figs. 1 and 2, protuberances 20 are in the shape of right circular cylinders of substantially uniform diameter "c," between about 0.5mm to about 1.5mm, a substantially uniform height "a" between about 1.5mm to about 2.5mm above surface 16 and substantially uniform spacing "b," between about 4mm to about 6mm, center-to-center. For some applications, the projection height "a" may range from about 1mm to 5mm or more, and spacing "b" may be between 3mm to more than 6mm. Preferred cylindrical protuberances 20 have axes "x" substantially perpendicular to the bottom surface 16.

Usage of the vessels of the present invention with coverslips is illustrated in Figs. 3, 4 and 5. Coverslips are generally glass and are commercially available in thicknesses from about 0.09mm to about 0.23mm. Coverslips generally are produced as circles with diameters about 12mm, 18mm, 22mm and 25mm; as squares with 18mm, 22mm and 25mm sides; and as rectangles with sides ranging from 11 ml by 22mm to 48mm by 60mm. Fig. 3 schematically illustrates a cross-section of coverslip 22 in position on protuberances 20 of several shapes. Protuberances 20 support coverslip 22 substantially parallel to bottom surface 16. Fig. 4 illustrates manipulation of coverslip 22 with forceps when the coverslip is supported on the protuberances. As shown in Fig. 5, vessels of the present invention are useful with coverslips of various shapes and sizes. Depending upon the size of the vessel and the coverslip selected, the receptacle may accommodate one or more coverslips.

Many cells form confluent monolayers on clean glass surfaces coated with protein. The glass coverslips are widely available and quite uniform. In current practice to form a cell mono-culture, cleaned glass coverslips are coated with protein, often polylysine, allowed to dry and placed in a laboratory culture vessel, often a flat circular open dish with straight sidewalls having a flat bottom commonly called a "Petri" dish. A suspension of cells in a suitable, generally aqueous, medium is then introduced into the dish. The dish with the suspension is then incubated until a confluent layer of cells develops on the coverslip. The coverslip is then removed from the vessel, generally with forceps, and the desired analysis conducted. Since both the coverslip and the vessel bottom surface both are substantially flat, the coverslips often are quite difficult to lift from the surface because of surface tension effects of the aqueous medium between the coverslip and the bottom surface. Often the confluent cell layer is disrupted or the coverslip broken in manipulations with forceps.

To conduct a similar mono-culture using preferred vessel 10 of the present invention, preferred protuberances 20 support coverslip 22 above and substantially parallel to the receptacle bottom. Referring to Figs. 3, 4 and 5, protein coated coverslip 22 is placed in receptacle 12 of vessel 10 and the suspension of cells in medium is added in similar fashion to the procedure followed above. When the operator removes coverslip 22 with forceps, as shown in Fig. 4, there is substantially less adhesion between coverslip 22 and bottom surface 16 of the receptacle because protuberances 20 support coverslip 22 above and substantially parallel to bottom surface 16, thus allowing the coverslip to be easily removed for analysis.

In the example of a co-culture cited above in the background, Culturing Nerve Cells, Banker and Goslin, MIT Press, (1991), the cited protocol gives a method for preparing a hippocampal neuron culture in the presence of glial cells using coverslips and culture vessels. The cited protocol describes applying wax beads to coverslips to support the coverslips above the surface of the vessel. To conduct a similar co-culture using vessel 10 of the present invention, the operator coats coverslip 22 with polylysine as is done in the protocol, but since protuberances 20 support coverslip 22 above surface 16 of the receptacle, the need for the operator to add wax beads to the coverslip is eliminated. In the co-culture of neurons in the presence of glial cells, the operator prepares a confluent layer of glial cells on bottom surface 16 of receptacle 10 with protuberances 20. The neurons are concurrently cultured on the surface of a coverslip as reported above for the mono-culture, then the coverslip with the neurons on one surface is transferred to the vessel with the glial cells on its bottom surface and placed with the neuron covered surface facing the confluent layer of glial cells on the vessel bottom. A suitable medium is then added and the vessel incubated. Preferred protuberances 20 support the neuron covered coverslip above and substantially parallel to the bottom surface of the receptacle, in close proximity to the glial cells but substantially without direct contact between the neurons on the coverslip and the glial cells on the receptacle bottom surface. Thus, by use of vessel 10 of the present invention, the operator can concentrate attention on the techniques of cell culture manipulations or the analysis of the cells and not have to spend time developing and practicing the technique of applying wax droplets to coverslips, substantially reducing the time necessary to get to the actual co-culture of the cells and increasing the efficiency of the laboratory.

A cylindrical shape is preferred for protuberances 20 because this shape minimizes the tendency of the cellular monolayer to cover the protuberance. A shape such as a hemisphere would tend to be covered by the confluent cellular layer. Preferably, protuberances 20 are integrally formed with surface 16 of the receptacle. Vessel 10 may be round square. rectangular or any other shape desired for a particular application. In this description, for brevity, Figs. 1, 2, 4 and 5 show vessel 10 in the form of a Petri dish. Other shapes are considered to be within the scope of the present invention.

Vessel 10 of the present invention may be formed in any of the normal sizes currently used for laboratory culture vessels. The vessel may be supplied with or without a fitted cover. Additionally, vessel 10 may be of one shape and receptacle 12 may be of another. The vessel may be formed from glass, polystyrene, polycarbonate, polyamide, polyester, and the like. Preferably, vessel 10 is formed by injection molding from crystalline polystyrene so that it is substantially transparent. It is preferred that the protuberances be formed integrally on the bottom surface from the same material used to mold the vessel.

Preferably vessel 10 is sealed in a package, shown in phantom as reference character 40 in Fig. 1, formed from material substantially resistant to the passage of microorganisms and exposed to conditions capable of rendering any microorganisms inside the package non-viable. Vessel 10 preferably is supplied with a cover 42 included in package 40. Suitable packaging materials include, but are not limited to, paper, non-wovens and polymeric films. Suitable treatments for rendering microorganisms non-viable, include ethylene oxide exposure and exposure to ionizing radiation. In the protocol described in the cited reference, the authors include resterilization of the coverslips after the addition of wax droplets because of the possibility of contamination during the handling required by the addition of the wax droplets. Vessel 10 of the present invention makes it unnecessary to resterilize the coverslips. When preferred vessel 10 is supplied sterilized in a package, the operator may simply remove vessel 10 from package 40 and proceed to starting the desired cell culture.

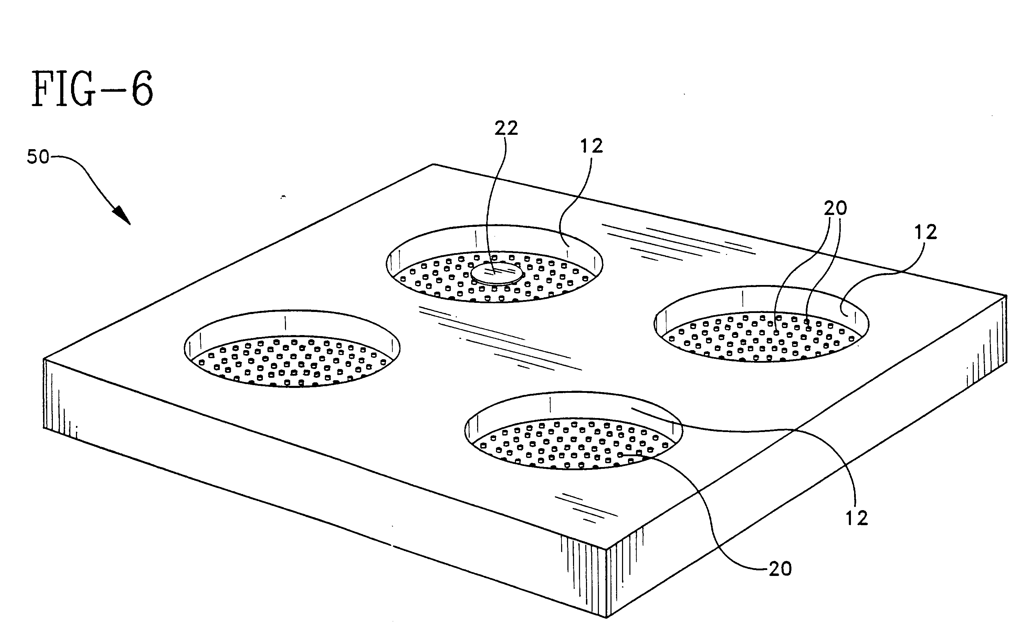

An alternative embodiment of the present invention is illustrated in Fig. 6. In this embodiment, the structure of the receptacle in the vessel is substantially similar to the vessel shown in Figs. 1-5. In this embodiment, a plurality of circular receptacles 12 is incorporated into a rectangular multi-well plate 50. For particular applications, the number of receptacles incorporated into a multi-well plate may range from two to more than one hundred. In Fig. 6, plate 50 has four receptacles 12. A coverslip is positioned in one receptacle for reference.

The vessel of the present invention provides a practitioner of cell mono-culture and co-culture a more efficient usage of coverslips for culturing cells. When used in a mono-culture, the protuberances support the coverslips above the bottom surface allowing easier manipulation of the coverslips, substantially reducing incidence of coverslip breakage due to their sticking to the vessel bottom. Additionally, for co-culture usage, the protuberances support coverslips having a first population of cells in close proximity to a second population of cells grown on the bottom of the vessel without direct contact between the cell populations. The coverslip with the first population then is easily removed from the vessel for study.

## Claims

1. A vessel for culturing cells comprising:
an open receptacle having a bottom with a surface; and
a multiplicity of protuberances projecting upwardly from said surface.

2. The vessel of claim 1 wherein said protuberances project upwardly between about one mm to about three mm, said protuberances having a spacing about three mm to about six mm therebetween, so that when a coverslip is placed on said bottom of said vessel, the coverslip is supported above and substantially parallel to said surface.

3. The vessel of claim 1 wherein said protuberances comprise a plurality of substantially parallel ridges with a height between about 1mm to about 5mm and a width between about 0.5mm to about 1.5mm said ridges spaced apart between about 3mm to about 6mm.

4. The vessel of claim 2 wherein said protuberances comprise a first plurality of substantially parallel segmented ridges disposed in a first direction and a second plurality of segmented ridges disposed in a second direction intersecting said first plurality of ridges, said intersecting ridges thereby forming a multiplicity of cross-shaped protuberances.

5. An open vessel for culturing cells comprising a receptacle having a substantially flat bottom having an inside surface, said inside surface of said flat bottom having a multiplicity of upward protuberances thereon.

6. The vessel of claim 5 wherein said protuberances have shapes selected from the group consisting of cones, frustrums of cones. cylinders, hemispheres and cubes.

7. The vessel of claim 5 wherein said protuberances comprise cylinders having a substantially uniform diameter between about 0.5mm to about 1.5mm with a substantially uniform height between about 1.5mm to about 2.5mm above said surface, said cylinders being substantially uniformly spaced between about 4mm to about 6mm center-to-center so that when a coverslip is placed on said bottom of said vessel the coverslip is supported above and substantially parallel to said bottom.

8. The vessel of claim 7 wherein said cylinders are right circular cylinders with axes substantially perpendicular to said bottom.

9. The vessel of claim 5 wherein said vessel comprises a multiwell plate having a plurality of receptacles therein.

10. The vessel of claim 5 further including a lid.

11. The vessel of claim 5 wherein said protuberances are integrally formed with said surface, said vessel being formed from a resin selected from the group consisting of polystyrene, polycarbonate, polypropylene, polyamide and polyester.

12. The vessel of claim 5 wherein said vessel is substantially transparent, formed from crystalline polystyrene, sealed in a package formed from materials substantially resistant to the passage of microorganisms and having been exposed to conditions substantially rendering any microorganisms therein nonviable.
